(19)
Europäisches
Patentamt

European
Patent Office

Office européen
des brevets

(11) **EP 4 209 548 A1**

(12) **EUROPEAN PATENT APPLICATION**
published in accordance with Art. 153(4) EPC

(43) Date of publication:
**12.07.2023 Bulletin 2023/28**

(21) Application number: **21864386.4**

(22) Date of filing: **02.09.2021**

(51) International Patent Classification (IPC):
*C08L 33/26* (2006.01)      *C08F 2/44* (2006.01)
*C12M 1/00* (2006.01)      *C08K 3/34* (2006.01)

(52) Cooperative Patent Classification (CPC):
**C08F 2/44; C08K 3/34; C08L 33/26; C12M 1/00**

(86) International application number:
**PCT/JP2021/032217**

(87) International publication number:
**WO 2022/050331 (10.03.2022 Gazette 2022/10)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **04.09.2020   JP 2020149039**

(71) Applicant: **FUJIFILM Corporation
Tokyo 106-8620 (JP)**

(72) Inventors:
- **SAKURAI Naoto
  Sakura-shi, Chiba 285-8668 (JP)**
- **NAKAGUMA Hirohide
  Tokyo 103-8233 (JP)**
- **TAKADA Tetsuo
  Sakura-shi, Chiba 285-8668 (JP)**

(74) Representative: **Hoffmann Eitle
Patent- und Rechtsanwälte PartmbB
Arabellastraße 30
81925 München (DE)**

(54) **ORGANIC-INORGANIC COMPLEX LIQUID DISPERSION AND METHOD FOR PRODUCING SAME**

(57)    An object is to provide an organic-inorganic composite dispersion liquid which is used for forming a dried coating film of a cell culture base material and with which a dried coating film exhibiting good film-forming properties or water resistance can be formed. The object is achieved by an organic-inorganic composite dispersion liquid comprising organic-inorganic composite particles (X) which are formed of a polymer (P) of a monomer including a (meth)acrylamide-based monomer (a) and one or more inorganic materials (B) selected from a water-swellable clay mineral or silica in a three-dimensional network and are dispersed in an aqueous medium (C), in which an average particle diameter of the organic-inorganic composite particles (X) is 100 nm or more.

EP 4 209 548 A1

**Description**

**BACKGROUND OF THE INVENTION**

1. Field of the Invention

The present invention relates to an organic-inorganic composite dispersion liquid containing organic-inorganic composite particles (X) which are formed of a polymer (P) of (meth)acrylamide-based monomer (a) and a water-swellable clay mineral (B) in a three-dimensional network, and are dispersed in an aqueous medium (C).

2. Description of the Related Art

An organic-inorganic composite dispersion liquid containing organic-inorganic composite particles (X), which are formed of a polymer (P) of a monomer including a (meth)acrylamide-based monomer (a) and one or more inorganic materials (B) selected from a water-swellable clay mineral or silica in a three-dimensional network, and are dispersed in an aqueous medium (C), has been known (for example, see JP2011-144236A).
In addition, in JP2011-144236A, it is disclosed that a dried coating film of the organic-inorganic composite particles (X), which is obtained by drying the organic-inorganic composite dispersion liquid, is used as a cell culture base material.

**SUMMARY OF THE INVENTION**

Incidentally, in the cell culture base material having the dried coating film formed by applying and drying the above-described organic-inorganic composite particles (X), as a base material for culturing cells, that is, as a base material which serves as a scaffold for cultured cells, it is required not to deteriorate under culture conditions. For example, in a case of culturing cells, various culture media are added to the cell culture base material, and in this case, the cell culture base material is required to have water resistance so as not to cause deterioration such as dissolution of the cell culture base material.
However, it cannot be said that the cell culture base material having the dried coating film obtained by drying the organic-inorganic composite dispersion liquid disclosed in JP2011-144236A has sufficient performance in terms of film-forming properties or water resistance, and there is room for improvement.
Therefore, an object of the present invention is to provide an organic-inorganic composite dispersion liquid which is used for forming a dried coating film of a cell culture base material and with which a dried coating film exhibiting good film-forming properties or water resistance can be formed.
As a result of intensive research to achieve the above-described object, the present inventor has found that, by polymerizing a (meth)acrylamide-based monomer (a) under specific reaction conditions, organic-inorganic composite particles (X) having a large particle diameter can be obtained, and in a case where a dried coating film is formed using an organic-inorganic composite dispersion liquid in which the organic-inorganic composite particles (X) having a large particle diameter are dispersed in an aqueous medium (C), the dried coating film has excellent film-forming properties or water resistance, thereby achieving the above-described object. Accordingly, the present invention has been completed.
That is, the present invention includes the following aspects.

[1] An organic-inorganic composite dispersion liquid comprising:

organic-inorganic composite particles (X) which are formed of a polymer (P) of a monomer including a (meth)acrylamide-based monomer (a) and one or more inorganic materials (B) selected from a water-swellable clay mineral or silica in a three-dimensional network and are dispersed in an aqueous medium (C),
in which an average particle diameter of the organic-inorganic composite particles (X) is 100 nm or more.

[2] The organic-inorganic composite dispersion liquid according to [1],
in which a mass ratio ((B)/(P)) of the one or more inorganic materials (B) selected from a water-swellable clay mineral or silica to the polymer (P) is in a range of 0.01 to 10.
[3] The organic-inorganic composite dispersion liquid according to [1] or [2],
in which the (meth)acrylamide-based monomer (a) is one or more (meth)acrylamide-based monomers selected from an N-substituted (meth)acrylamide derivative, an N,N-disubstituted (meth)acrylamide derivative, or N,N′ -methylenebisacrylamide.
[4] The organic-inorganic composite dispersion liquid according to any one of [1] to [3],

in which the water-swellable clay mineral is at least one clay mineral which delaminates into 1 to 10 layers in the aqueous medium (C), the at least one clay mineral being selected from water-swellable hectorite, water-swellable montmorillonite, water-swellable saponite, or water-swellable synthetic mica, and the silica is water-dispersible colloidal silica.

[5] A dried coating film of the organic-inorganic composite particles (X), which is obtained by drying the organic-inorganic composite dispersion liquid according to any one of [1] to [4].

[6] A cell culture base material comprising:
the dried coating film according to [5] on a surface of the cell culture base material.

[7] The cell culture base material according to [6], further comprising:

a support,
in which the dried coating film is laminated on the support.

[8] A method for producing an organic-inorganic composite dispersion liquid in which organic-inorganic composite particles (X) are dispersed in an aqueous medium (C), the method comprising:

a step of forming the organic-inorganic composite particles (X) by mixing a (meth)acrylamide-based monomer (a), one or more inorganic materials (B) selected from a water-swellable clay mineral or silica, a polymerization initiator (D), and the aqueous medium (C), and polymerizing the (meth)acrylamide-based monomer (a),
in which a concentration (% by mass) of the one or more inorganic materials (B) selected from a water-swellable clay mineral or silica in the aqueous medium (C) is in a range represented by Expression (1) or Expression (2), and
in the case of polymerizing the (meth)acrylamide-based monomer (a), the (meth)acrylamide-based monomer (a) is polymerized by setting a temperature condition of the aqueous medium (C) to be equal to or higher than a lower critical solution temperature (LCST) of the organic-inorganic composite dispersion liquid,

$$\text{Expression (1)} \quad \text{in a case where Ra} < 0.19,$$

$$\text{concentration of inorganic material (B) (\% by mass)} < 12.4\text{Ra} + 0.05$$

$$\text{Expression (2)} \quad \text{in a case where Ra} \geq 0.19,$$

$$\text{concentration of inorganic material (B) (\% by mass)} < 0.87\text{Ra} + 2.17$$

(in the expressions, the concentration of the inorganic material (B) (% by mass) is a value obtained by dividing a mass of the inorganic material (B) by a total mass of the aqueous medium (C) and the inorganic material (B) and multiplying the resultant by 100, and Ra is a mass ratio ((B)/(P)) of the inorganic material (B) to a polymer (P)).

[9] The method for producing an organic-inorganic composite dispersion liquid according to [8],
in which the temperature condition of the aqueous medium (C) is higher than the LCST of the organic-inorganic composite dispersion liquid by 10°C or higher.

[10] The method for producing an organic-inorganic composite dispersion liquid according to [8] or [9],
in which in the case of polymerizing the (meth)acrylamide-based monomer (a), the (meth)acrylamide-based monomer (a) is polymerized by a polymerization reaction by heating or irradiation with ultraviolet rays.

[11] The method for producing an organic-inorganic composite dispersion liquid according to any one of [8] to [10],
in which in the case of polymerizing the (meth)acrylamide-based monomer (a), the temperature condition of the aqueous medium (C) is set to 45°C to 55°C, potassium peroxodisulfate and N,N,N',N'-tetramethylethylenediamine are used as a polymerization initiator or a catalyst, and the (meth)acrylamide-based monomer (a) is polymerized by a polymerization reaction by heating.

According to the present invention, it is possible to provide an organic-inorganic composite dispersion liquid which is used for forming a dried coating film of a cell culture base material and with which a dried coating film exhibiting good film-forming properties or water resistance can be formed.

## DESCRIPTION OF THE PREFERRED EMBODIMENTS

Hereinafter, the present invention will be described in detail. The description of configuration requirements described below is an example for explaining the present invention, and the present invention is not limited thereto.

In addition, a notation of "(meth)acrylamide-based monomer" indicates both an acrylamide-based monomer and a methacrylamide-based monomer.

(Organic-inorganic composite dispersion liquid)

In the organic-inorganic composite dispersion liquid, organic-inorganic composite particles (X) are dispersed in an aqueous medium (C).

The organic-inorganic composite particles (X) are formed of a polymer (P) and an inorganic material (B) in a three-dimensional network.

The polymer (P) is obtained by polymerizing a monomer including a (meth)acrylamide-based monomer (a).

The inorganic material (B) includes one or more selected from a water-swellable clay mineral or silica.

An average particle diameter of the organic-inorganic composite particles (X) in the organic-inorganic composite dispersion liquid is 100 nm or more.

Hereinafter, each component constituting the organic-inorganic composite dispersion liquid will be described in detail.

<(Meth)acrylamide-based monomer (a)>

The (meth)acrylamide-based monomer (a) can be suitably used as long as the polymer (P) thereof can interact with the inorganic material (B) to form organic-inorganic composite particles, and among these, N-substituted (meth)acrylamide derivatives such as N-methyl(meth)acrylamide, N-ethyl(meth)acrylamide, and N-isopropyl(meth)acrylamide, N,N-disubstituted (meth)acrylamide derivatives such as N,N-dimethyl(meth)acrylamide and (meth)acryloylmorpholine, N,N'-methylenebisacrylamide, or N,N-dimethylaminoethyl (meth)acrylate is preferably used. Two or more kinds of these monomers (a) can be mixed and used.

Among the above-described monomers (a), in a case of a polymer (P) having a lower critical solution temperature (LCST), for example, in a case of using N-isopropyl acrylamide, it is possible to obtain a dispersion liquid having an energy-saving dimming function such that transparency of the dispersion liquid changes according to the environmental temperature.

<Polymer (P)>

The polymer (P) is obtained by polymerizing a monomer including the above-described (meth)acrylamide-based monomer (a).

In addition to the above-described (meth)acrylamide-based monomer (a), in order to impart a balance of hydrophilicity and hydrophobicity to the organic-inorganic composite body and to add a functional group, other polymerization monomers can be used in combination as necessary. That is, the polymer (P) can be obtained by polymerizing the above-described (meth)acrylamide-based monomer (a) and other polymerization monomers.

For example, as the other polymerization monomers, a (meth)acrylic monomer having an anionic group such as a sulfone group and a carboxyl group, a (meth)acrylic monomer having a cationic group such as a quaternary ammonium group, a (meth)acrylic monomer having a zwitterionic group with a quaternary ammonium group and a phosphate group, a (meth)acrylic monomer having an amino acid residue with a carboxyl group and an amino group, a (meth)acrylic monomer having a sugar residue, a (meth)acrylic monomer having a hydroxyl group, polyethylene glycol, a (meth)acrylic monomer having a polypropylene glycol chain, an amphipathic (meth)acrylic monomer having both a hydrophilic chain such as polyethylene glycol and a hydrophobic group such as a nonylphenyl group, polyethylene glycol diacrylate, methoxyethyl (meth)acrylate, or the like can be used in combination.

<Inorganic material (B)>

The inorganic material (B) used in the present invention is one or more inorganic materials (B) selected from a water-swellable clay mineral or silica.

Examples of the water-swellable clay mineral include swellable clay minerals which can delaminate into layers, and a clay mineral which can swell and be uniformly dispersed in water or a mixed solution of water and an organic solvent is preferably used, and an inorganic clay mineral which can be uniformly dispersed in water to be a molecular form (single layer) or to be a near level thereof is particularly preferably used.

In addition, the water-swellable clay mineral is preferably a clay mineral which delaminates into 1 to 10 layers in the aqueous medium (C).

Specific examples of the water-swellable clay mineral include water-swellable hectorite containing sodium as an interlayer ion, water-swellable montmorillonite, water-swellable saponite, and water-swellable synthetic mica. These clay minerals may be mixed and used.

Examples of the silica ($SiO_2$) used in the present invention include colloidal silica, and colloidal silica which can be uniformly dispersed in an aqueous solution and has a particle diameter of 10 nm to 500 nm is preferably used, and colloidal silica having a particle diameter of 10 to 50 nm is particularly preferably used.

By using the inorganic material (B), stability of the organic-inorganic composite dispersion liquid and coating film forming ability are good, and a dried coating film having excellent antifogging properties is obtained.

In the present invention, from the viewpoint of obtaining organic-inorganic composite particles (X) having a more desired particle diameter, it is more preferable to use the water-swellable clay mineral than the silica.

<Organic-inorganic composite particles (X)>

The organic-inorganic composite particles (X) are formed of the above-described polymer (P) and the above-described inorganic material (B) in a three-dimensional network.

A mass ratio ((B)/(P)) of the polymer (P) to the inorganic material (B) is preferably 0.01 to 10, more preferably 0.03 to 5, and particularly preferably 0.05 to 3.

In a case where the mass ratio ((B)/(P)) is within the range, the obtained organic-inorganic composite dispersion liquid has good stability, and a smooth coating film having strong adhesiveness to the base material is easily obtained.

An average particle diameter of the organic-inorganic composite particles (X) dispersed in the organic-inorganic composite dispersion liquid is 100 nm or more as described above. As a result, the average particle diameter of the organic-inorganic composite particles (X) in the present invention is as large as 100 nm or more. Accordingly, a dried coating film obtained by coating with the organic-inorganic composite dispersion liquid according to the embodiment of the present invention, in which the organic-inorganic composite particles having such a large particle diameter are dispersed, has excellent film-forming properties and water resistance, as shown in Examples.

In order to form the organic-inorganic composite particles (X) having the particle diameter specified in the present invention, in consideration of types and amounts of the polymerization catalyst and the polymerization initiator used in polymerizing the monomers including the (meth)acrylamide-based monomer (a), types and amounts of respective components constituting the organic-inorganic composite particles (X), an amount of the aqueous medium (C), and the like, it is necessary to polymerize the monomer including the (meth)acrylamide-based monomer (a) under specific polymerization temperature conditions. A method for producing the organic-inorganic composite particles (X), including the polymerization temperature conditions, will be described in detail in the section of (Method for producing organic-inorganic composite dispersion liquid) later.

The average particle diameter of the organic-inorganic composite particles (X) is 100 nm or more, and is preferably 150 nm or more and more preferably 200 nm or more. In a case of being 100 nm or more, film-forming properties and water resistance is obtained. In a case of being 200 nm or more, it is assumed that the film-forming properties are further improved.

In addition, in consideration of dispersibility, transparency of the coating film, and the like, the average particle diameter of the organic-inorganic composite particles (X) is preferably 1,000 nm or less, more preferably 800 nm or less, and still more preferably 700 nm or less.

[Method of measuring average particle diameter of organic-inorganic composite particles (X)]

The average particle diameter of the emulsion particles can be obtained by taking a 50% median diameter measured by a dynamic light scattering method, for example, a volume-based 50% median diameter measured by a dynamic light scattering particle size distribution analyzer (for example, a dynamic light scattering particle size distribution analyzer LB-550 manufactured by HORIBA, Ltd.) as the average particle diameter.

<Aqueous medium (C)>

The aqueous medium (C) used in the production method according to the embodiment of the present invention is not particularly limited as long as it is a medium which can include the (meth)acrylamide-based monomer (a), the inorganic material (B), and the like to obtain an organic-inorganic composite dispersion liquid having good physical properties. For example, the aqueous medium (C) may be water or an aqueous solution including a solvent miscible with water and/or other compounds, can further include a preservative, an antibacterial agent, a coloring agent, a fragrance, an enzyme, protein, sugars, amino acids, cells, DNAs, salts, water-soluble organic solvents, a surfactant, a polymer compound, a leveling agent, or the like.

<Characteristics of organic-inorganic composite dispersion liquid>

By performing a gel fraction measurement test using a dried film obtained by applying and drying the organic-inorganic composite dispersion liquid according to the embodiment of the present invention, water resistance of the dried film can be evaluated.

Since the dried film obtained by using the organic-inorganic composite dispersion liquid according to the embodiment of the present invention exhibits a high gel fraction, it can be said that the dried film is excellent in film-forming properties and water resistance.

The gel fraction of the dried film obtained by using the organic-inorganic composite dispersion liquid according to the embodiment of the present invention is preferably 80% or more, more preferably 90% or more, and still more preferably 95% or more.

[Method of measuring gel fraction of organic-inorganic composite dispersion liquid]

The gel fraction of the dried film obtained by using the organic-inorganic composite dispersion liquid according to the embodiment of the present invention can be obtained by producing a thin film and measuring a weight loss rate of the thin film, as shown in Examples.

The organic-inorganic composite dispersion liquid is cast on a PP tray of approximately 15 cm $\times$ 20 cm so as to have a thickness of 10 to 500 $\mu$m, dried at room temperature, and then dried at 130°C for 1 hour to obtain a dry coating film (hereinafter, referred to as a dry coating film).

Approximately 0.2 g of the obtained dry coating film is wrapped in a 200-mesh stainless wire mesh, dried at 130°C for 2 hours, and weighed. Thereafter, the whole mesh is put into water and left at approximately 23°C for 20 to 24 hours. After taking out the mesh, the film is dried in a hot air dryer at 130°C for 2 hours, and weighed. From each measured weight, the weight loss rate before and after being allowed to stand in water is examined. This value is defined as the gel fraction (%).

It can be said that, as the value of the gel fraction (%) is higher, the dry coating film has high water resistance, and in a case where the dry coating film is used in a dry culture base material, elution due to water or a culture medium is unlikely to occur.

(Method for producing organic-inorganic composite dispersion liquid)

A method for producing an organic-inorganic composite dispersion liquid in which the organic-inorganic composite particles (X) are dispersed in the aqueous medium (C) will be described in detail below.

The method for producing an organic-inorganic composite dispersion liquid according to the embodiment of the present invention includes a step of forming the organic-inorganic composite particles (X) by mixing the (meth)acrylamide-based monomer (a), one or more inorganic materials (B) selected from a water-swellable clay mineral or silica, a polymerization initiator (D), and the aqueous medium (C), and polymerizing the (meth)acrylamide-based monomer (a).

Examples of a preferred aspect of the method for producing an organic-inorganic composite dispersion liquid according to the embodiment of the present invention include a method for producing an organic-inorganic composite dispersion liquid including the following steps (I) and (II):

(I) step of mixing the (meth)acrylamide-based monomer (a), one or more inorganic materials (B) selected from a water-swellable clay mineral or silica, a polymerization initiator (D), and the aqueous medium (C); and
(II) step of polymerizing the (meth)acrylamide-based monomer (a).

Hereinafter, each step will be described in detail.

<Step (I): mixing step>

By mixing the (meth)acrylamide-based monomer (a), one or more inorganic materials (B) selected from a water-swellable clay mineral or silica, and the polymerization initiator (D), the components are dissolved or uniformly dispersed in the aqueous medium (C).

The (meth)acrylamide-based monomer (a), the one or more inorganic materials (B) selected from a water-swellable clay mineral or silica, and the aqueous medium (C), which are mixed components, are as described in each item of (Organic-inorganic composite dispersion liquid).

<<Polymerization initiator (D)>>

As the polymerization initiator (D) used in the production method according to the embodiment of the present invention, a known radical polymerization initiator can be appropriately selected and used. An initiator having water dispersibility and being uniformly included in the entire system is preferably used. Specific examples of the polymerization initiator include water-soluble peroxides such as potassium peroxodisulfate and ammonium peroxodisulfate, water-soluble azo compounds such as VA-044, V-50, and V-501 (all manufactured by Wako Pure Chemical Industries, Ltd.), a photopolymerization initiator (Irg184 or the like), and a mixture of $Fe^{2+}$ and hydrogen peroxide.

As a catalyst, N,N,N',N'-tetramethylethylenediamine and the like, which are tertiary amine compounds, are preferably used. However, the catalyst does not necessarily have to be used.

On the other hand, the photopolymerization initiator is suitably used because it is not easily affected by oxygen inhibition and has a high polymerization rate. Specific examples thereof include acetophenones such as p-tert-butyltrichloroacetophenone, benzophenones such as 4,4'-bisdimethylaminobenzophenone, ketones such as 2-methylthioxanthone, benzoin ethers such as benzoinmethyl ether, $\alpha$-hydroxyketones such as hydroxycyclohexylphenyl ketone, phenylglyoxylates such as methylbenzoylformate, and metallocenes.

The above-described photopolymerization initiator is preferably water-insoluble. The term "water-insoluble" as used herein means that an amount of the polymerization initiator dissolved in water is 0.5% by mass or less. By using the water-insoluble polymerization initiator, the initiator is more likely to be present in the vicinity of the inorganic material (B), the number of initiation reaction points from the vicinity of the inorganic material (B) is increased, the obtained organic-inorganic composite body has a narrow particle size distribution, and stability of the dispersion liquid is high, which is preferable. There is no problem in using any of the polymerization methods in terms of film-forming properties and water resistance, but in terms of particle diameter, for example, a large particle diameter tends to be formed by performing a thermal polymerization using potassium peroxodisulfate than an UV photopolymerization of Irg184, and a larger particle diameter tends to be formed by using potassium peroxodisulfate and an amine-based catalyst such as N,N,N',N'-tetramethylethylenediamine. The UV polymerization is difficult to mass produce in batch type, and the mass production is easier and more preferable to the thermal polymerization.

It is preferable to add, to the aqueous medium (C), a solution obtained by dissolving the above-described photopolymerization initiator in a solvent (F) compatible with the aqueous medium (C). By this method, the photopolymerization initiator can be more uniformly dispersed, and composite particles having a more uniform particle diameter are obtained.

As the solvent (F) of the present invention, a water-soluble solvent capable of dissolving the water-insoluble photopolymerization initiator, or the (meth)acrylamide-based monomer (a) or another water-soluble acrylic monomer (a') can be used. Examples of the water-soluble solvent include amides such as dimethylacetamide and dimethylformamide, alcohols such as methanol and ethanol, dimethylsulfoxide, and tetrahydrofuran. These solvents may be mixed and used.

In addition, examples of the (meth)acrylamide-based monomer (a) or water-soluble acrylic monomer (a'), which can be used as the solvent (F), include polypropylene glycol diacrylates such as tripropylene glycol diacrylate, polyethylene glycol diacrylates, polypropylene glycol acrylates such as pentapropylene glycol acrylate, polyethylene glycol acrylates, methoxyethyl acrylate, methoxypolyethylene glycol acrylates such as methoxytriethylene glycol acrylate, nonylphenoxy polyethylene glycol acrylates, N-substituted acrylamides such as dimethylacrylamide, hydroxyethyl acrylate, and hydroxypropyl acrylate. Two or more kinds of these acrylic monomers can be mixed and used.

The water-soluble solvent referred to herein is preferably a solvent capable of dissolving 50 g or more of the water-insoluble photopolymerization initiator in 100 g of water. Within this range, dispersibility of the water-insoluble photopolymerization initiator (D) in the aqueous medium (C) is good, the particle diameter of the obtained organic-inorganic composite particles is likely to be uniform, and the stability of the dispersion liquid is good.

A mass ratio (D)/(F) of the photopolymerization initiator (D) to the solvent (F) in the solution in which the water-insoluble photopolymerization initiator (D) is dissolved in the solvent (F) is preferably 0.001 to 0.1 and more preferably 0.01 to 0.05. In a case of being 0.001 or more, a sufficient amount of radicals generated by irradiation with energy rays can be obtained, so that the polymerization reaction can be suitably progressed, and in a case of being 0.1 or less, the initiator does not substantially cause color development or odor, and cost can be reduced.

In any case of the above acrylic monomer (a') and the water-soluble solvent, an addition amount of the solution in which the photopolymerization initiator (D) is dissolved in the solvent (F) is preferably 0.1% by mass to 5% by mass, and more preferably 0.2% by mass to 2% by mass with respect to the total mass of the (meth)acrylamide-based monomer (a), the inorganic material (B), the aqueous medium (C), the polymerization initiator (D), and the solvent (F). In a case where the addition amount is 0.1% by mass or more, the polymerization is sufficiently initiated, and in a case of being less than 5% by mass, it is possible to reduce problems such as the generation of odor due to an increase in polymerization initiator in the organic-inorganic composite particles (X), and re-aggregation of the photopolymerization initiator once dispersed, so that a uniform organic-inorganic composite dispersion liquid can be obtained, which is preferable.

In addition, in the method for producing an organic-inorganic composite dispersion liquid according to the embodiment of the present invention, a concentration (% by mass) of the one or more inorganic materials (B) selected from a water-swellable clay mineral or silica in the aqueous medium (C) shows a range represented by Expression (1) or Expression (2).

$$\text{Expression (1)} \quad \text{in a case where Ra} < 0.19,$$

$$\text{concentration of inorganic material (B) (\% by mass)} < 12.4\text{Ra} + 0.05$$

$$\text{Expression (2)} \quad \text{in a case where Ra} \geq 0.19,$$

$$\text{concentration of inorganic material (B) (\% by mass)} < 0.87\text{Ra} + 2.17$$

(in the expressions, the concentration of the inorganic material (B) (% by mass) is a value obtained by dividing a mass of the inorganic material (B) by a total mass of the aqueous medium (C) and the inorganic material (B) and multiplying the resultant by 100, and Ra is a mass ratio ((B)/(P)) of the inorganic material (B) to the polymer (P))

By satisfying Expression (1) or Expression (2), granular organic-inorganic composite body is uniformly dispersed in the aqueous medium, and a good emulsion can be formed without gelation. In a case where the concentration (% by mass) of the inorganic material (B) with respect to the aqueous medium is the above-described range or more, gelation of the entire reaction system occurs due to the polymerization, or the organic-inorganic composite dispersion liquid is non-uniform, so that a good organic-inorganic composite dispersion liquid cannot be produced.

<Step (II): step of polymerizing (meth)acrylamide-based monomer (a)>

In polymerizing the (meth)acrylamide-based monomer (a) in the method for producing an organic-inorganic composite dispersion liquid according to the embodiment of the present invention, in a case where a monomer having a lower critical solution temperature (LCST) is used for the polymer, the polymerization is preformed in a state in which a temperature condition of the aqueous medium (C) is set to be equal to or higher than the lower critical solution temperature (LCST) of the organic-inorganic composite dispersion liquid.

In polymerizing the (meth)acrylamide-based monomer (a), in a case where the polymerization temperature is set to a high temperature as equal to or higher than the lower critical solution temperature (LCST) of the organic-inorganic composite dispersion liquid, as described above, the present inventors have found that organic-inorganic composite particles (X) having a large particle diameter of 100 nm or more are obtained. This is much larger than the particle diameter of the known organic-inorganic composite particles in the related art. The reason why the organic-inorganic composite particles (X) having such a large particle diameter are obtained is presumed as follows. In the first place, since the organic-inorganic composite particles (X) obtained in the present invention have temperature-responsive properties, it is considered that physical properties of the polymer during the polymerization are different between polymerization at elevated temperature and polymerization at room temperature or lower. Therefore, in the case of polymerization, by raising the temperature to be equal to or higher than LCST, it is considered that an interaction between the polymers occurs, resulting in formation of organic-inorganic composite particles which are easily entangled and polymerized while entangled, so that organic-inorganic composite particles having a large particle diameter are obtained. In addition, by raising the temperature, it is considered that radical migration and crosslinking occur, which cause a three-dimensional polymer to undergo polymerization like a branched polymer, so that the particle diameter can be large.

In addition, it is considered that the reason why the dried coating film which is obtained by using the organic-inorganic composite dispersion liquid having the organic-inorganic composite particles having such a large particle diameter is excellent in film-forming properties and water resistance is that crosslinking points of the organic-inorganic composite particles increase and a crosslinking density increase, which enhance the water resistance in film formation.

Although the lower critical solution temperature (LCST) of the organic-inorganic composite dispersion liquid varies depending on LCST of the polymer P and the types of respective components constituting the organic-inorganic composite particles (X), the LCST can be determined by the following measurement.

[Method of measuring LCST of organic-inorganic composite dispersion liquid]

As the LCST, using a temperature-variable ultraviolet-visible spectrophotometer, a temperature of a cell including the organic-inorganic composite dispersion liquid is observed while increasing the temperature at a heating rate of 10 °C/60 min, and a cloudiness start temperature (LCST) of the organic-inorganic composite dispersion liquid is measured. For example, a transmittance at 600 nm is measured, and a portion where the transmittance is 50% or less and is cloudy is read.

As the polymerization temperature condition in the polymerization of the (meth)acrylamide-based monomer (a), that is, the temperature condition of the aqueous medium (C) is equal to or higher than the LCST temperature of the organic-inorganic composite dispersion liquid, preferably higher than the LCST by 10°C or higher.

In addition, more specifically, the temperature condition of the aqueous medium (C) in the polymerization of the (meth)acrylamide-based monomer (a) is preferably 35°C or higher and more preferably 40°C or higher. In addition, as the temperature condition, in order to prevent side reactions in water, it is preferably 90°C or lower, more preferably 80°C or lower, and still more preferably 70°C or lower.

Therefore, the polymerization temperature condition in the polymerization of the (meth)acrylamide-based monomer (a) is preferably 35°C to 90°C, more preferably 40°C to 80°C, and still more preferably 40°C to 70°C.

In a dispersion liquid (E) including the (meth)acrylamide-based monomer (a), the inorganic material (B), the polymerization initiator (D), and the aqueous medium (C), the method for polymerizing the (meth)acrylamide-based monomer (a) is not particularly limited, and may be a polymerization method by heating or a polymerization method by irradiation with energy rays (for example, irradiation with ultraviolet rays).

However, from the viewpoint of obtaining organic-inorganic composite particles (X) having a larger particle diameter, a polymerization by heating is more preferable.

Examples of the polymerization method in a case of using the above-described photopolymerization initiator include irradiation with energy rays, and for example, electron beam, $\gamma$-ray, X-ray, ultraviolet ray, visible light, or the like can be used. Among these, from the viewpoint of convenience of the apparatus and handling, it is preferable to use ultraviolet rays. An intensity of the ultraviolet rays to be irradiated is preferably 10 to 500 mW/cm$^2$, and an irradiation time is generally approximately 0.1 seconds to 200 seconds.

In a normal radical polymerization by heating, oxygen acts as an inhibitor of the polymerization, but in the present invention, it is not necessary to prepare the solution and perform the polymerization by irradiation with energy rays in an oxygen-blocked atmosphere, and it is possible to prepare the solution and perform the polymerization by irradiation with energy rays in an air atmosphere. However, in some cases, it is possible to further increase the polymerization rate by performing the irradiation with ultraviolet rays in an inert gas atmosphere, which is desirable.

It is preferable that the polymerization method is carried out in an inert gas atmosphere regardless of whether the polymerization method is based on heating or on irradiation with energy rays (for example, irradiation with ultraviolet rays).

For example, in a case where the (meth)acrylamide-based monomer (a) is polymerized by the polymerization reaction by heating, preferred examples of the method for producing an organic-inorganic composite dispersion liquid according to the embodiment of the present invention include a production method according to the following embodiments.

Examples thereof include a method for producing an organic-inorganic composite dispersion liquid, in which, in polymerizing the (meth)acrylamide-based monomer (a), the temperature condition of the aqueous medium (C) is set to be equal to or higher than the lower critical solution temperature (LCST) of the organic-inorganic composite dispersion liquid (for example, 45°C to 50°C), a polymerization initiator or a catalyst (for example, potassium peroxodisulfate (KSP), or potassium peroxodisulfate (KSP) and N,N,N',N'-tetramethylethylenediamine (TEMED)) is used, and the (meth)acrylamide-based monomer (a) is polymerized by a polymerization reaction by heating.

Examples of a still more preferred embodiment include a method for producing an organic-inorganic composite dispersion liquid, in which the (meth)acrylamide-based monomer (a) and a pure water (C) are stirred and mixed at room temperature, and then the inorganic material is added; under a nitrogen atmosphere, the inorganic material is dispersed in the mixed solution heated to 50°C; next, potassium peroxodisulfate (KSP) and N,N,N',N'-tetramethylethylenediamine (TEMED) are added thereto; and under a nitrogen atmosphere, the (meth)acrylamide-based monomer (a) is reacted in the mixed solution at 50°C for 0.5 to 3 hours to form the polymer (P). As a result, it is possible to produce an organic-inorganic composite dispersion liquid in which the organic-inorganic composite particles (X), which are formed of the polymer (P) and the inorganic material (B) in a three-dimensional network, are dispersed in the aqueous medium (C).

For example, in a case where the (meth)acrylamide-based monomer (a) is polymerized by the polymerization reaction by irradiation with ultraviolet rays, preferred examples of the method for producing an organic-inorganic composite dispersion liquid according to the embodiment of the present invention include a production method according to the following embodiments.

Examples thereof include a method for producing an organic-inorganic composite dispersion liquid, in which, in polymerizing the (meth)acrylamide-based monomer (a), the temperature condition of the aqueous medium (C) is set to be equal to or higher than the lower critical solution temperature (LCST) of the organic-inorganic composite dispersion liquid (for example, 45°C to 50°C), a photopolymerization initiator (for example, 1-hydroxycyclohexylphenylketone (Irg184) is used, and the (meth)acrylamide-based monomer (a) is polymerized by a polymerization reaction by irradiation with ultraviolet rays.

Examples of a still more preferred embodiment include a method for producing an organic-inorganic composite dispersion liquid, in which the inorganic material (B), the (meth)acrylamide-based monomer (a), and the pure water (C) are mixed, and these components are dispersed in the mixed solution heated to 45°C to 50°C under a nitrogen atmosphere; next, 1-hydroxycyclohexylphenylketone (Irg184) dissolved in methanol is added thereto; and under a nitrogen atmosphere, the (meth)acrylamide-based monomer (a) is reacted by irradiation with ultraviolet rays in the mixed solution at 45°C to 50°C for 3 minutes to form the polymer (P). As a result, it is possible to produce an organic-inorganic composite dispersion liquid in which the organic-inorganic composite particles (X), which are formed of the polymer (P) and the inorganic material (B) in a three-dimensional network, are dispersed in the aqueous medium (C).

(Use of organic-inorganic composite dispersion liquid)

<Dried coating film>

By drying the organic-inorganic composite dispersion liquid according to the embodiment of the present invention, a transparent dried coating film having favorable flexibility and mechanical properties can be obtained. The dried coating film may be in a state (coating film) of being attached to a support (base material), or may be in a form of an independent film without the support. A thickness of the dried coating film can be optionally adjusted depending on the intended use, and a thickness of the dried coating film as the independent film is preferably 0.01 mm to 2 mm because it is easy to handle. Within this range, strength of the dried coating film is sufficient, it is easy to handle, and it is easy to manufacture a dried coating film having high surface smoothness. In addition, a thickness of the dried coating film in a state being attached to the support is not particularly limited, but it can be handled suitably as long as the thickness thereof is 0.001 $\mu$m or more, and it is preferable to be 0.005 $\mu$m or more considering the range without influence of the support.

The organic-inorganic composite dispersion liquid according to the embodiment of the present invention may be used as it is as a coating material, or may be used after undergoing a purification step such as washing with water. In addition, for the purpose of imparting functionality such as coating properties, surface smoothness of the dried coating film, dimming performance, and antifogging properties, a leveling agent, a surfactant, a polymer compound, a colorant, or the like may be further added and used to the organic-inorganic composite dispersion liquid.

A laminate can be produced by applying the organic-inorganic composite dispersion liquid according to the embodiment of the present invention onto a support and forming a dried coating film. In a case of applying the organic-inorganic composite dispersion liquid onto the support, the organic-inorganic composite dispersion liquid can be applied in a patterned manner having a specific shape as necessary. Examples of a method of applying the organic-inorganic composite dispersion liquid onto the support in a patterned manner include a printing method in which the dispersion liquid is applied to a plate with a pattern and then transferred to the support, and a method of pattern coating in which portions not to be coated on the support are covered in advance and the covered portions are removed after coating, and a method of applying the dispersion liquid using an inkjet printer method.

The dried coating film obtained by applying and drying the organic-inorganic composite dispersion liquid according to the embodiment of the present invention can be used as a cell culture base material, a warming film, or the like due to its temperature response characteristics.

<Cell culture base material>

By applying the organic-inorganic composite dispersion liquid according to the embodiment of the present invention onto a support (for example, a polystyrene container), drying the organic-inorganic composite dispersion liquid according to the embodiment of the present invention, washing as necessary, and drying in a state of being attached to the support, it can be used as a cell culture base material with good adhesiveness and proliferation. In addition, by selecting the polymer (P), cells do not adhere to a culture surface, and the cell culture base material can be used as a culture base material capable of culturing in a float state (cell low adhesiveness).

A shape of the cell culture base material according to the present invention is not particularly limited as long as the cells can be cultured and the cultured cells can be easily detached by a low-temperature treatment. Examples thereof include a film-shaped base material, a dish-shaped base material, a bottle-shaped base material, a tube-shaped base material, a thread-shaped or rod-shaped base material having a thickness of 5 nm to 5 mm, a bag-shaped base material, a multi-well plate-shaped base material, a microchannel-shaped base material, a porous membrane-shaped or network-shaped base material (for example, TRANSWELL and a cell strainer), and a spherical-shaped base material having a particle diameter of preferably 10 to 2,000 $\mu$m and more preferably 100 to 500 $\mu$m.

The cell culture base material according to the present invention may be used alone, but is preferably used as a cell culture equipment in which the base material is formed on the support. This is because, in a case of using as the cell culture equipment, convenience of transportation, storage, and the like is excellent, and the cell culture equipment can be used as it is as a culture container or a culture carrier.

A material of the support to be used in the present invention is not particularly limited as long as the cell culture base material can be sufficiently adhered thereto, cells can be cultured on the cell culture base material adhered, and the cultured cells can be easily detached by a low-temperature treatment. For example, a styrene-based resin such as polystyrene; a polyolefin-based resin such as polypropylene; a polyurethane-based resin; a polycarbonate; polyethylene terephthalate (PET); a polysulfone-based resin; a fluororesin, a polysaccharide natural polymer such as cellulose; an inorganic material such as glass or ceramic; or a metallic material such as stainless steel or titanium is suitably used.

A shape of the support is not particularly limited as long as it can be a support for the cell culture base material according to the present invention. Examples thereof include a film-shaped support, a membrane-shaped support, a plate-shaped support, a spherical-shaped support, a polygonal-shaped support, a rod-shaped support, a dish-shaped support, a bottle-shaped support, a tube-shaped support, a needle or thread-shaped support, a fiber-shaped support, a bag-shaped support, a multi-well plate-shaped support, a microchannel-shaped support, a porous membrane-shaped support, and a network-shaped support (for example, TRANSWELL and a cell strainer). A shape combining these shapes may be used, or an irregularly shaped support which does not have a specific shape may be used.

Furthermore, the cell culture base material according to the present invention may be integrated with the support and used as the cell culture equipment, or may be peeled off from the support and used alone.

Examples

Hereinafter, the present invention will be described in more detail with reference to Examples, but the scope of the present invention is not limited to Examples.

(Example 1)

[Preparation of reaction solution (E) including (meth)acrylamide-based monomer (a), water-swellable clay mineral (B), photopolymerization initiator (D), and aqueous medium (C)]

1.13 g of N-isopropyl acrylamide (NIPAM) (manufactured by KOHJIN Film & Chemicals Co., Ltd.) as a (meth)acrylamide-based monomer (a), 0.08 g of Laponite XLG (manufactured by Rockwood Additives Ltd.) as a clay mineral (B), and 40 g of ultrapure water as an aqueous medium (C) were mixed, and these components were heated to 45°C to 50°C in a nitrogen atmosphere.

Next, 200 μL of an Irg184 solution as a water-insoluble photopolymerization initiator (D) was mixed thereto to prepare a reaction solution (E1). Here, the Irg184 solution is a methanol solution having a concentration of Irg184 of 2% by mass.

[Production of dispersion liquid of organic-inorganic composite body X]

Under a nitrogen atmosphere, the above-described reaction solution (E1) heated to 45°C was irradiated with ultraviolet rays at 365 nm with an ultraviolet intensity of 40 mW/cm$^2$ for 3 minutes while stirring the reaction solution (E1) with a magnetic stirrer to polymerize the (meth)acrylamide-based monomer (a), thereby producing an organic-inorganic composite dispersion liquid (1) (hereinafter, also referred to as a dispersion liquid (1)) of an organic-inorganic composite body X, which was slightly cloudy.

Concentrations of Ra and the inorganic material (B) in this reaction system are as follows.

$$Ra = 0.07, \text{ concentration of inorganic material (B) (\% by mass)} = 0.20 \, (\%) < 12.4Ra + 0.05 = 0.92$$

The container including the above-described dispersion liquid (1) was placed in a water tank, and in a case where a cloudiness start temperature (LCST) of the dispersion liquid was measured while raising the temperature of the water tank from room temperature at a heating rate of 10 °C/60 min, the LCST of the dispersion liquid (1) was approximately 33°C.

In the production of the above-described dispersion liquid (1), a reaction conversion rate of the (meth)acrylamide-based monomer (a) was 97%.

Here, the reaction conversion rate was calculated by quantifying a residual amount of the monomer by HPLC and converting it from the charged amount.

In a case where a particle diameter of the organic-inorganic composite particles in the above-described dispersion liquid (1) was measured using a dynamic light scattering particle size distribution analyzer (for example, a dynamic light scattering particle size distribution analyzer LB-550 manufactured by HORIBA, Ltd.), an average particle diameter was 132 nm.

With the organic-inorganic composite dispersion liquid (1) obtained in Example 1, the organic-inorganic composite dispersion liquid was cast on a PP tray of approximately 15 cm × 20 cm so as to have a thickness of 50 μm, dried at room temperature, and then dried at 130°C for 1 hour to obtain a dry coating film.

Approximately 0.2 g of the obtained dry coating film was wrapped in a 200-mesh stainless wire mesh, dried at 130°C for 2 hours, and weighed. Thereafter, the whole mesh was put into water and left at approximately 23°C for 20 hours. After taking out the mesh, the film was dried in a hot air dryer at 130°C for 2 hours, and weighed. From each measured weight, a weight loss rate before and after being allowed to stand in water was examined. This value was defined as a gel fraction (%).

As a result, the gel fraction of the dry culture base material having the dried coating film of the organic-inorganic composite dispersion liquid (1) was 93%.

The evaluation results of the organic-inorganic composite dispersion liquid (1) of Example 1 are shown in Table 1 below.

(Examples 2 and 3)

Reaction solutions (E2) and (E3) were prepared in the same manner as in Example 1, except that the content of the water-swellable clay mineral (B) in Example 1 was changed as shown in Table 1 below.

Here, concentrations and Ra of the inorganic material (B) in the reaction system in the reaction solution (E2) are as follows.

$$Ra = 0.14, \text{ concentration of inorganic material (B) (\% by mass)} = 0.40\ (\%) < 12.4Ra + 0.05 = 1.79$$

Here, concentrations and Ra of the inorganic material (B) in the reaction system in the reaction solution (E3) are as follows.

Ra = 0.28, concentration of inorganic material (B) (% by mass) = 0.80 (%) < 12.4Ra + 0.05 = 3.52

Evaluation results of the organic-inorganic composite dispersion liquids (2) and (3) of Examples 2 and 3 are shown in Table 1 below.

(Example 4)

[Preparation of reaction solution (E) including (meth)acrylamide-based monomer (a), water-swellable clay mineral (B), polymerization initiator (D), and aqueous medium (C)]

1.13 g of N-isopropyl acrylamide (manufactured by KOHJIN Film & Chemicals Co., Ltd.) as a (meth)acrylamide-based monomer (a) and 40 g of pure water as an aqueous medium (C) were mixed and stirred at room temperature. Next, 0.16 g of Laponite XLG (manufactured by Rockwood Additives Ltd.) as a clay mineral (B) was mixed, and these components were heated to 50°C under a nitrogen atmosphere and stirred for 10 minutes.

Next, 0.82 mL of a potassium peroxodisulfate (KPS) solution as a polymerization initiator (D) was mixed to prepare a reaction solution (E4). Here, the KPS solution is an aqueous solution having a concentration of KPS of 2% by mass.

[Production of dispersion liquid of organic-inorganic composite body X]

Under a nitrogen atmosphere, the above-described reaction solution (E4) was heated to 70°C and then reacted for 0.5 hours while stirring the reaction solution (E4) with a magnetic stirrer to polymerize the (meth)acrylamide-based monomer (a), thereby producing an organic-inorganic composite dispersion liquid (4) of an organic-inorganic composite body X, which was slightly cloudy.

Evaluation results of the organic-inorganic composite dispersion liquid (4) of Example 4 are shown in Table 1 below.

(Example 5)

[Preparation of reaction solution (E) including (meth)acrylamide-based monomer (a), water-swellable clay mineral (B), polymerization initiator (D), and aqueous medium (C)]

1.13 g of N-isopropyl acrylamide (manufactured by KOHJIN Film & Chemicals Co., Ltd.) as a (meth)acrylamide-based monomer (a) and 40 g of pure water as an aqueous medium (C) were mixed and stirred at room temperature. Next, 0.08 g of Laponite XLG (manufactured by Rockwood Additives Ltd.) as a clay mineral (B) was mixed, and these components were heated to 50°C under a nitrogen atmosphere and stirred for 10 minutes.

Next, 0.41 mL of a potassium peroxodisulfate (KPS) solution as a polymerization initiator (D) and 0.5 mL of an N,N,N',N'-tetramethylethylenediamine (TEMED) solution as a catalyst were mixed to prepare a reaction solution (E5). Here, the KPS solution is an aqueous solution having a concentration of KPS of 2% by mass, and the TEMED solution is an aqueous solution having a concentration of TEMED of 2% by mass.

[Production of dispersion liquid of organic-inorganic composite body X]

Under a nitrogen atmosphere, the above-described reaction solution (E5) was heated to 50°C and then reacted for 1 hour while stirring the reaction solution (E5) with a magnetic stirrer to polymerize the (meth)acrylamide-based monomer (a), thereby producing an organic-inorganic composite dispersion liquid (5) of an organic-inorganic composite body X, which was slightly cloudy.

Evaluation results of the organic-inorganic composite dispersion liquid (5) of Example 5 are shown in Table 1 below.

(Examples 6 and 7)

Reaction solutions (E6) and (E7) were prepared in the same manner as in Example 5, except that the content of the water-swellable clay mineral (B) in Example 5 was changed as shown in Table 1 below.

Evaluation results of the organic-inorganic composite dispersion liquids (6) and (7) of Examples 6 and 7 are shown in Table 1 below.

(Comparative Example 1)

Reaction solutions (comparative E1 to comparative E4) were prepared in the same manner as in Example 1, except that, in Example 1, the content of each constituent component and the heating temperature of the reaction solution in the polymerization conditions were changed as shown in Table 1 below.

Evaluation results of the organic-inorganic composite dispersion liquids (comparative 1 to comparative 4) of Comparative Examples 1 to 4 are shown in Table 1 below.

| | Constituent component | | | | Polymerization conditions | | | Reaction conversion rate (%) | Particle diameter (nm) | Gel fraction (%) |
|---|---|---|---|---|---|---|---|---|---|---|
| | Water-swellable clay mineral (B) (XLG) (g) | (Meth)acrylamide-based monomer(a) (NIPAM) (g) | Aqueous medium (C) (g) | Type of polymerization initiator (D) | Polymerization method | Polymerization temperature (°C) | Polymerization time | | | |
| Example 1 | 0.08 | 1.13 | 40 | Irg184 | UV | 45 | For 3 minutes | 97 | 132 | 93 |
| Example 2 | 0.16 | 1.13 | 40 | Irg184 | UV | 45 | For 3 minutes | 98 | 175 | 94 |
| Example 3 | 0.32 | 1.13 | 40 | Irg184 | UV | 45 | For 3 minutes | 95 | 125 | 97 |
| Example 4 | 0.16 | 1.13 | 40 | KPS | Thermal | 70 | For 0.5 hours | 99 | 176 | 96 |
| Example 5 | 0.08 | 1.13 | 40 | KPS/TEMED | Thermal | 50 | For 1 hour | 98 | 270 | 96 |
| Example 6 | 0.16 | 1.13 | 40 | KPS/TEMED | Thermal | 50 | For 1 hour | 98 | 305 | 97 |
| Example 7 | 0.32 | 1.13 | 40 | KPS/TEMED | Thermal | 50 | For 1 hour | 98 | 290 | 98 |
| Comparative Example 1 | 0 | 1.13 | 40 | Irg184 | UV | 15 | For 3 minutes | 98 | 14 | < 5 |
| Comparative Example 2 | 0.08 | 1.13 | 40 | Irg184 | UV | 15 | For 3 minutes | 98 | 53 | < 5 |
| Comparative Example 3 | 0.16 | 1.13 | 40 | Irg184 | UV | 15 | For 3 minutes | 97 | 74 | 71 |
| Comparative Example 4 | 0.08 | 1.13 | 40 | KPS/TEMED | Thermal | 15 | For 20 hours | 95 | 80 | 12 |

From Examples and Comparative Examples described above, in polymerizing the (meth)acrylamide-based monomer (a), in a case where the polymerization reaction was carried out at a high temperature, it was found that organic-inorganic composite particles (X) having a large particle diameter of 100 nm or more were obtained. A dried coating film obtained by applying and drying the organic-inorganic composite dispersion liquid in which the organic-inorganic composite particles (X) having a large particle diameter were dispersed exhibits a high gel fraction, and it was found that film-forming properties and water resistance were excellent.

In a case of using a thermal polymerization, the organic-inorganic composite particles (X) having a particle diameter of 300 nm or more could be obtained, and from the viewpoint of obtaining the organic-inorganic composite particles (X) having a larger particle diameter, the thermal polymerization was more preferred than the UV polymerization.

Therefore, according to the present invention, it is possible to provide an organic-inorganic composite dispersion liquid which is used for forming a dried coating film of a cell culture base material that can be adopted to a cell culture base material, and with which a dried coating film exhibiting good film-forming properties or water resistance can be formed.

**Claims**

1. An organic-inorganic composite dispersion liquid comprising:

   organic-inorganic composite particles (X) which are formed of a polymer (P) of a monomer including a (meth)acrylamide-based monomer (a) and one or more inorganic materials (B) selected from a water-swellable clay mineral or silica in a three-dimensional network and are dispersed in an aqueous medium (C),
   wherein an average particle diameter of the organic-inorganic composite particles (X) is 100 nm or more.

2. The organic-inorganic composite dispersion liquid according to claim 1,
   wherein a mass ratio ((B)/(P)) of the one or more inorganic materials (B) selected from a water-swellable clay mineral or silica to the polymer (P) is in a range of 0.01 to 10.

3. The organic-inorganic composite dispersion liquid according to claim 1 or 2,
   wherein the (meth)acrylamide-based monomer (a) is one or more (meth)acrylamide-based monomers selected from an N-substituted (meth)acrylamide derivative, an N,N-disubstituted (meth)acrylamide derivative, or N,N' -methylenebisacrylamide.

4. The organic-inorganic composite dispersion liquid according to any one of claims 1 to 3,

   wherein the water-swellable clay mineral is at least one clay mineral which delaminates into 1 to 10 layers in the aqueous medium (C), the at least one clay mineral being selected from water-swellable hectorite, water-swellable montmorillonite, water-swellable saponite, or water-swellable synthetic mica, and
   the silica is water-dispersible colloidal silica.

5. A dried coating film of the organic-inorganic composite particles (X), which is obtained by drying the organic-inorganic composite dispersion liquid according to any one of claims 1 to 4.

6. A cell culture base material comprising:

   the dried coating film according to claim 5 on a surface of the cell culture base material.

7. The cell culture base material according to claim 6, further comprising:

   a support,
   wherein the dried coating film is laminated on the support.

8. A method for producing an organic-inorganic composite dispersion liquid in which organic-inorganic composite particles (X) are dispersed in an aqueous medium (C), the method comprising:

   a step of forming the organic-inorganic composite particles (X) by mixing a (meth)acrylamide-based monomer (a), one or more inorganic materials (B) selected from a water-swellable clay mineral or silica, a polymerization initiator (D), and the aqueous medium (C), and polymerizing the (meth)acrylamide-based monomer (a),

wherein a concentration (% by mass) of the one or more inorganic materials (B) selected from a water-swellable clay mineral or silica in the aqueous medium (C) is in a range represented by Expression (1) or Expression (2), and in the case of polymerizing the (meth)acrylamide-based monomer (a), the (meth)acrylamide-based monomer (a) is polymerized by setting a temperature condition of the aqueous medium (C) to be equal to or higher than a lower critical solution temperature (LCST) of the organic-inorganic composite dispersion liquid,

$$\text{Expression (1)} \quad \text{in a case where Ra} < 0.19,$$

$$\text{concentration of inorganic material (B) (\% by mass)} < 12.4\text{Ra} + 0.05$$

$$\text{Expression (2)} \quad \text{in a case where Ra} \geq 0.19,$$

$$\text{concentration of inorganic material (B) (\% by mass)} < 0.87\text{Ra} + 2.17$$

(in the expressions, the concentration of the inorganic material (B) (% by mass) is a value obtained by dividing a mass of the inorganic material (B) by a total mass of the aqueous medium (C) and the inorganic material (B) and multiplying the resultant by 100, and Ra is a mass ratio ((B)/(P)) of the inorganic material (B) to a polymer (P)).

9. The method for producing an organic-inorganic composite dispersion liquid according to claim 8, wherein the temperature condition of the aqueous medium (C) is higher than the LCST of the organic-inorganic composite dispersion liquid by 10°C or higher.

10. The method for producing an organic-inorganic composite dispersion liquid according to claim 8 or 9, wherein in the case of polymerizing the (meth)acrylamide-based monomer (a), the (meth)acrylamide-based monomer (a) is polymerized by a polymerization reaction by heating or irradiation with ultraviolet rays.

11. The method for producing an organic-inorganic composite dispersion liquid according to any one of claims 8 to 10, wherein in the case of polymerizing the (meth)acrylamide-based monomer (a), the temperature condition of the aqueous medium (C) is set to 45°C to 55°C, potassium peroxodisulfate and N,N,N',N'-tetramethylethylenediamine are used as a polymerization initiator or a catalyst, and the (meth)acrylamide-based monomer (a) is polymerized by a polymerization reaction by heating.

<table>
<tr><td colspan="3" align="center">**INTERNATIONAL SEARCH REPORT**</td><td colspan="2">International application No.<br><br>**PCT/JP2021/032217**</td></tr>
</table>

**A.    CLASSIFICATION OF SUBJECT MATTER**

*C08L 33/26*(2006.01)i; *C08F 2/44*(2006.01)i; *C12M 1/00*(2006.01)i; *C08K 3/34*(2006.01)i
FI:    C08L33/26; C08K3/34; C08F2/44 A; C12M1/00 C

According to International Patent Classification (IPC) or to both national classification and IPC

**B.    FIELDS SEARCHED**

Minimum documentation searched (classification system followed by classification symbols)

C08L33/26; C08F2/44; C12M1/00; C08K3/34

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

**C.    DOCUMENTS CONSIDERED TO BE RELEVANT**

| Category* | Citation of document, with indication, where appropriate, of the relevant passages | Relevant to claim No. |
|---|---|---|
| X | JP 2014-7264 A (NIPPON CHEMICON CORP) 16 January 2014 (2014-01-16)<br>　　　claims, examples | 1-10 |
| A | | 11 |
| X | JP 2013-196942 A (KAWAMURA INSTITUTE OF CHEMICAL RESEARCH) 30 September 2013 (2013-09-30)<br>　　　claims, examples | 1-10 |
| A | | 11 |
| X | JP 2011-132345 A (KAWAMURA INSTITUTE OF CHEMICAL RESEARCH) 07 July 2011 (2011-07-07)<br>　　　claims, paragraph [0050], examples | 1-7 |
| A | | 8-11 |
| X | JP 2005-60448 A (FUJI XEROX CO LTD) 10 March 2005 (2005-03-10)<br>　　　claims, paragraph [0026], examples | 1-10 |
| A | | 11 |

☑ Further documents are listed in the continuation of Box C.        ☑ See patent family annex.

| | |
|---|---|
| *       Special categories of cited documents:<br>"A"    document defining the general state of the art which is not considered<br>　　　to be of particular relevance<br>"E"    earlier application or patent but published on or after the international<br>　　　filing date<br>"L"    document which may throw doubts on priority claim(s) or which is<br>　　　cited to establish the publication date of another citation or other<br>　　　special reason (as specified)<br>"O"    document referring to an oral disclosure, use, exhibition or other<br>　　　means<br>"P"    document published prior to the international filing date but later than<br>　　　the priority date claimed | "T"    later document published after the international filing date or priority<br>　　　date and not in conflict with the application but cited to understand the<br>　　　principle or theory underlying the invention<br>"X"    document of particular relevance; the claimed invention cannot be<br>　　　considered novel or cannot be considered to involve an inventive step<br>　　　when the document is taken alone<br>"Y"    document of particular relevance; the claimed invention cannot be<br>　　　considered to involve an inventive step when the document is<br>　　　combined with one or more other such documents, such combination<br>　　　being obvious to a person skilled in the art<br>"&"    document member of the same patent family |
| Date of the actual completion of the international search<br><br>**21 October 2021** | Date of mailing of the international search report<br><br>**02 November 2021** |
| Name and mailing address of the ISA/JP<br><br>**Japan Patent Office (ISA/JP)**<br>**3-4-3 Kasumigaseki, Chiyoda-ku, Tokyo 100-8915**<br>**Japan** | Authorized officer<br><br><br><br>Telephone No. |

Form PCT/ISA/210 (second sheet) (January 2015)

**INTERNATIONAL SEARCH REPORT**

International application No.

**PCT/JP2021/032217**

**C.    DOCUMENTS CONSIDERED TO BE RELEVANT**

| Category* | Citation of document, with indication, where appropriate, of the relevant passages | Relevant to claim No. |
|---|---|---|
| X | JP 2011-12107 A (KAWAMURA INST OF CHEM RES) 20 January 2011 (2011-01-20) claims, paragraphs [0009], [0018], [0029], examples | 1-4 |
| A | | 5-11 |
| X | JP 2003-96113 A (HYMO CORP) 03 April 2003 (2003-04-03) claims, paragraph [0043], examples | 1-3, 5-8, 10 |
| A | | 4, 9, 11 |
| A | JP 2005-110604 A (KAWAMURA INST OF CHEM RES) 28 April 2005 (2005-04-28) claims, paragraph [0025], examples | 1-11 |
| A | JP 2002-53762 A (KAWAMURA INST OF CHEM RES) 19 February 2002 (2002-02-19) claims, examples | 1-11 |

Form PCT/ISA/210 (second sheet) (January 2015)

**INTERNATIONAL SEARCH REPORT**
Information on patent family members

International application No.

**PCT/JP2021/032217**

| Patent document cited in search report | | | Publication date (day/month/year) | Patent family member(s) | | | Publication date (day/month/year) |
|---|---|---|---|---|---|---|---|
| JP | 2014-7264 | A | 16 January 2014 | (Family: none) | | | |
| JP | 2013-196942 | A | 30 September 2013 | WO | 2012/144332 | A1 | |
| JP | 2011-132345 | A | 07 July 2011 | (Family: none) | | | |
| JP | 2005-60448 | A | 10 March 2005 | (Family: none) | | | |
| JP | 2011-12107 | A | 20 January 2011 | (Family: none) | | | |
| JP | 2003-96113 | A | 03 April 2003 | (Family: none) | | | |
| JP | 2005-110604 | A | 28 April 2005 | (Family: none) | | | |
| JP | 2002-53762 | A | 19 February 2002 | US<br>claims, examples<br>US<br>EP | 2001/0049413<br><br>2004/0106722<br>1160286 | A1<br><br>A1<br>A1 | |

Form PCT/ISA/210 (patent family annex) (January 2015)

**REFERENCES CITED IN THE DESCRIPTION**

**Patent documents cited in the description**

- JP 2011144236 A